# EUROPEAN PATENT SPECIFICATION

(11) **EP 2 193 786 B1**
(45) Date of publication and mention of the grant of the patent: **23.04.2014**
(21) Application number: 09178433.0
(22) Date of filing: 08.12.2009
(51) Int. Cl.: A61K 9/00

(54) **Bolus and manufacturing process**
Bolus und Herstellungsverfahren
Bolus et procédé de fabrication

(30) Priority: 08.12.2008 GB 0822295
(43) Date of publication of application: 09.06.2010
(73) Proprietor: Agrimin Limited, Humberside Airport Kirmington North Lincolnshire DN39 6YH (GB)
(72) Inventor: Bennison, James John, Dr., Lincolnshire LN8 2DB (GB)
(74) Representative: Thomson, Craig Richard

(56) References cited:
- GB-A- 2 408 453
- US-A- 4 166 107
- US-A- 5 252 561
- US-A- 5 869 083
- US-A1- 2002 037 317

## Description

The present invention relates to boluses for administering beneficial substances to animals, and to methods of making same.

In order to maintain health, domesticated animals are often required to regularly ingest active substances, such as dietary supplements or therapeutic substances. Due to the difficulty of administering such active substances to animals, easily swallowable boluses that include the active substance can be made for ingestion by the animal. Bolus administration is particularly useful for ruminant animals (such as cows, sheep, goats and deer) as the boluses can be made in order to be retained in the rumen or reticulum of the animal, thereby enabling a sustained release of the active substances from the boluses into the gastrointestinal tract of the animal over a period of time as the boluses slowly disintegrate in a controlled manner.

Boluses are generally composed of a binder and a densifier. The binder is a substance that provides shape and body to the bolus, being able to cohere all components of the bolus into a distinct package. Thus, waxes, resins, or mixtures thereof, are often used as binders. Densifiers provide weight to the bolus. Therefore, densifiers are often metals. The weight of a densifier in a bolus enhances the ability for the animals to swallow the bolus, and can enable the bolus to be retained within the rumen or reticulum of a ruminant animal.

The most common method of making boluses, particular intra-ruminal boluses, is by extrusion. It has been found by the inventors, however, that forming boluses (particularly those with a high level of densifier) is difficult, as the pressure acting on the pre-solidified mixture at the dye head induces separation of the densifier from the other components of the bolus. The other conventional methods for manufacturing boluses are by compression moulding or tableting; both of which require compression of the composition of densifier and binder by the application of pressures well exceeding 2 bar. Again, however, it was found that this compression results in the separation of densifier and other components of the bolus.

The separation of densifier from the other components has been found by the inventors to result in a bolus that provides poor sustained release profiles, and one that is particularly brittle around portions of high density densifier (in worst cases resulting in a mass that is not capable of being cohered by the binder to form a distinct bolus).

US Patent Application No. 4 166 107 A describes boluses made from carnauba wax and barium sulphate. Production of such boluses is described as including the steps of melting monostearin and carnauba wax before blending the mixture with further ingredients including barium sulphate and blending until a creamy mixture is obtained. The resulting creamy mixture is poured into a mould. US Patent Application No. 2002/037317 A1 discloses the manufacture of a bolus prepared by melting carnauba wax and di[alpha] tocopheryl acetate and zinc powder so as to form a mixture that is transferred to a mould and preferably compressed. British Patent Application No. 2 408 453 A describes, a mixture of molten wax binder and zinc that is prepared before being passed into a mould or extruded through a dye. US Patent Application No. 5 869 083 A describes a bolus formed by introducing layers of sugar granules and copper oxide particles into a mould prior to heating the mould so as to melt the sugar; the subsequent cooling of the contents of the mould forming a solidified bolus.

Following extensive experimentation the inventors have found a new process for forming boluses.

### Summary of the Invention

In a first aspect of the present invention, there is provided a process for manufacturing a bolus, wherein the process comprises the steps of:-
a. introducing a densifier and a binder into a mould without the densifier and binder having been mixed prior to step a;
b. mixing the contents of the mould, which comprise the densifier and the binder, by vibration;
c. solidifying the contents of the mould so as to form a bolus, wherein the binder comprises a resin or a wax and is fluid during steps a. and b.

Surprisingly, it has been found that this process produces boluses with improved release profiles for active substances provided within the bolus, when compared with boluses prepared using conventional processes. Furthermore, It has been found that this process can eliminate the need for pre-mixing of the densifier and binder of the bolus (the vibration being sufficient for mixing thereof), thereby providing a simpler and so more cost efficient process.

Consequently, in one embodiment of the present invention the densifier and the binder are not mixed together before being introduced to the mould. The densifier and binder may therefore be added separately, sequentially or simultaneously to the mould. When the densifier is added sequentially with the binder to the mould, the densifier may be added to the mould before the binder. It has been found that mixing of the densifier and binder may then take place by vibration in the mould.

Conventional pre-mixing of densifier and binder requires the post-mixing action of urging the mix into the mould. The applicants have found that such an action is difficult to achieve without at least partial separation of the densifier from the binder, which can result in an incomplete transport of densifier into the mould. This can result in an incorrect proportion of densifier and binder in the mould, and ultimately an incorrect proportion of densifier and binder in the final bolus (which can have deleterious effects on the release profiles and integrity of the bolus).

The densifier may be made of any substance that may add weight to the bolus, and that would be safely consumed by an animal in the quantities used in the bolus. The densifier may be an inert densifier, in which case the densifier is not included to provide any active substance itself. Alternatively, the densifier may be an "active" densifier, and so will directly provide an active substance itself (e.g. zinc). Boluses with active densifiers may additionally comprise a non-densifier active substance. Not wishing to be restricted further, but in the interests of clarity, the densifier may comprise or consist of a metal or metal compound. For example:- zinc, iron, manganese, copper, cobalt, barium, or the like, or any combination thereof. Metal compounds may be for example:- metal oxides, metal sulphates, or the like, or any combination thereof. Preferably the metal compounds are those derived from the aforementioned metals. In order to ease the addition of the densifier to the mixture, and to ensure that the densifier is easily dispersed through the bolus on vibration, the densifier is preferably particulate. In one embodiment of the present invention, the particles are not rods or filaments, e.g. the diameters of each particle does not vary by more than 25% (i.e. the particles are roughly spherical). Preferably the particles of densifier have a diameter of less than 1000µm, 750 µm, 500 µm or 250 µm. It has been found that smaller particles easily agglomerate during production of the bolus and can inhibit proper bolus formation. Accordingly, it is preferred that no more than about 40% of the particles have a diameter of less than 45 µm. Relatively large particles, on the other hand, have been found to have good flow properties and so it is preferred that at least 50% of densifier particles have a diameter of from 45 µm to 150 µm. In fact, the inventors have identified that using a particulate densifier with a wide distribution of particle size is preferred. Thus, the densifier may be provided in particulate form where 5% or less of the particles have diameter of greater than 150 µm, from 50 to 80% of the particles have a diameter of from 45 µm to 150 µm, and less than 40% of the particles have a diameter of less than 45 µm. It is particularly preferred that the densifier and the binder are not mixed together before being introduced to the mould when at least 50%, 60%, 70%, 80%, 90% or more, or 100% of the particles of densifiers used in the process of the present invention have diameters of less than 45 µm and/or greater than 150 µm. It has been found that mixtures of densifier and binder with densifiers within such ranges are the most vulnerable to separation during the action of passing the mixture to the mould, particularly because they have poor flow properties and must therefore be forced into the moulds under pressure.

From 60 to 95%, 70 to 95%, 80 to 95, or 85 to 95 % by weight of the bolus is preferably densifier. The particles of densifier may be of non-uniform shape (i.e. irregular in shape, as opposed to the regular spherical particle shape that can be achieved by spray drying).

The binder may as defined by claim 1 provide shape and body to the bolus, is capable of cohering all components of the bolus into a distinct package, and that would be safely consumed by an animal in the quantities used in the bolus. The densifier preferably has a melting temperature that is higher than the body temperature of the animal in which the bolus is to be used, thereby preventing melting of the bolus in use and so preventing uncontrolled release of active agent. Thus, the densifier preferably has a melting temperature that is greater than 20, 25, 30, 35, 37 or 40 °C. The melting temperature of the binder helps to dictate the hardness of the bolus and so the erosion rate of the bolus when in the animal. The inventors have found that melting temperatures of less than 80°C are preferred. Not wishing to be restricted further, but in the interests of clarity, the binder comprises or consists of a resin or wax (of plant or mineral origin), or a combination thereof. The binder is preferably a non-sugar binder. For example, the binder may be paraffin wax, carnauba wax, bees wax, or the like, or any combination thereof. The optimum amount of binder in the bolus has been found to vary as a function of the particle size distribution of the densifier.

In one embodiment of the present invention the binder is used in an amount required to form a bolus that has a substantially homogeneous dispersal of densifier throughout the bolus. Therefore, no excess binder is used. In alternative embodiment of the present invention, an excess of binder is used. Preferably an excess of binder results in the production of a bolus wherein a first region of the bolus comprises densifier (preferably homogeneously mixed throughout the binder), and a second region comprises binder and in which densifier is absent (as is described in more detail below). The second region may represent up to 1% of the total weight of the bolus. In practice, it has been found that filling the mould with sufficient binder to cover the densifier in the mould, prior to step b, provides an excess of binder in the bolus. Preferably, the amount of binder used is the minimum amount of binder required to cover all the densifier in the mould, prior to step b (i.e. an amount of binder that just covers the densifier, and no more).

Thus in one embodiment of the present invention, step a includes the step of applying the minimum amount of binder to the mould that covers all the densifier in the mould.

Not wishing to be restricted further, but in the interest of clarity, the boluses can be formed using from 12-14% by weight of binder in the bolus, preferably using the aforementioned particulate densifier, and preferably in the aforementioned amounts thereof.

In one embodiment of the present invention, the combination of the densifier and binder are chosen so as to produce a bolus with a density that encourages the retention of the bolus in the rumen or reticulum of the animal to be treated by the bolus. For example, the bolus has a density of 2.7 g/ml or greater, or 3.0 g/ml or greater. Boluses preferably have a density of less than 6.0 g/ml. It has been found that for retention in cattle the density is preferably greater than 2.7 g/ml, and preferably greater than 3.0 g/ml for sheep.

Not wishing to be bound by theory, but in the interests of clarity, after noticing the advantages derived from vibrating the contents of the mould the inventors have hypothesised that the vibration enhances the packing geometry of the densifier, thereby ensuring the preferred substantial homogeneous and tight dispersal of the densifier through the bolus. Before vibration, the variously sized particles of densifier filing the mould along with the binder fill the available space to produce a particle bed that is in static equilibrium owing to the interaction of gravitational and adhesive/cohesive forces. By vibration of the bed, particles can be mobilized so that if the vibration is stopped, the bed is once more in static equilibrium but occupies a different spatial volume than before. In general, such geometric rearrangements result in a transition from loosely packed particles to more tightly packed ones.

The vibration of the contents of the mould can be achieved in a number of ways. For example, the vibration may be applied directly to the mould, for example to the external wall of the mould such that the vibrator does not contact the composition to form the bolus. Alternatively, the vibrator maybe applied to the surface of the contents of the mould or inserted into the contents of the mould. When the vibrator is applied to the surface of the contents of the mould, the vibrator can be a compactor, but preferably does not cover more than 50% of the surface area of the surface of the contents of the mould and/or applies less than 2 bar pressure to the surface. During the period of vibration the contents of the mould should be substantially fluid. The frequency of vibration may be at about 50 HZ.

In a preferred embodiment of the present invention, the vibration establishes a substantially homogeneous dispersion of the densifier throughout the bolus (or at least a substantially homogeneous dispersion through a first region of the bolus, and no densifier in a second).

In order to ease the transfer of the binder to the mould, and the action of vibration on the contents of the mould, it is preferred that the binder is a fluid during steps a and b. This may be achieved in a number of ways. For example, the binder may be heated prior to or during steps a and/or b such that the binder is molten during steps a and b. The binder is then preferably cooled during step c such that the binder solidifies. Alternatively, the binder includes a solvent that renders the binder fluid during steps a and b. The solvent is preferably removed (e.g. by evaporation; passively or on the application of heat) during step c so such "curing" results in solidifying of the binder.

Preferably the contents of the mould are not compressed. However, compression may enhance the surface finish of the bolus. Preferably, if compression is used in the process, the compression does not exceed 2 bar, which has been found to produce a good surface finish whilst minimising the risk of separation of the densifier. Accordingly, the process preferably includes a step of applying a pressure of between 0 and 2 bar to the contents of the mould. The step of applying pressure may be carried out between step a and step c, and preferably after step b. The ability to use a process that requires no, or low, pressures means that once can use rubber or plastics moulds.

Step a. may include the introduction of a therapeutic agent to the mould. The therapeutic agent may be the densifier and/or a separate substance added to the bolus. The therapeutic agent may be any agent used to treat the animal to ingest the bolus, or a dietary supplement for the animal. Not wishing to be restricted further, but in the interest of clarity, the therapeutic agent may comprise:- selenium, cobalt, iodine, manganese, copper, zinc, vitamins, lipids, biotin, amino acids, anthelminitic agents, antibacterial agents, growth promoters, hormones, coccidiostats, flukicides, or the like, or any combination thereof.

Step a. may include the introduction to the mould of additives that provide lubrication, enhance the surface finish of the bolus, and/or alter the physical properties of the binder (such as melting temperature and/or crystal structure). Suitable additives are microcrystalline wax, stearic acid, or an oil (such as ground nut oil or sunflower oil), or the like, or any combination thereof.

In a further embodiment of the present invention, the process further comprises the step of heating the mould and its contents. The mould and contents is preferably heated to a temperature that is above that of the melting temperature of the binder, preferably more than 25°C above the melting temperature of the binder. Not wishing to be restricted further, but in the interest of clarity, the temperature may be from 85 to 100°C. The heating of the mould and contents may be carried out after step a. but prior to step b. It has been found that such a step is able to eliminate, or substantially eliminate, air bubbles from the mixture of densifier and binder. The mould and contents may be heated for less than 30, 20 or 15 minutes.

In a further aspect of the present invention, there is provided a bolus as defined by claim 11 manufactured by the process described in the first aspect of the present invention.

If yet a further aspect of the present invention as defined in claim 11 there is provided a bolus comprising densifier and binder, wherein a first region of the bolus comprises densifier homogeneously mixed throughout the binder, and a second region comprising binder and in which densifier is absent. Preferably, the second region forms a film on a portion of the surface area of the bolus. The portion of the surface of the bolus on which the film is provided is preferably one that faces a consistent direction over that portion (i.e. is substantially a single flat surface of the bolus). Most preferably, the portion is the upward facing surface of the bolus when the bolus is in the mould. The film is preferably not more than 3, 4, 5, or 6 mm thick. This may be achieved by processing the bolus in accordance with the first aspect of the present invention, when there is an excess of binder used. Not wishing to be bound by theory, but in the interests of clarity, the inventors believe that the densifier particles are initially loosely packed in the mould but following vibration they become more tightly packed and occupy a smaller volume. Because there is insufficient space between the particles, and because binder is fluid and less dense than densifier, the excess binder rises in the mould during the process. It then forms a layer at the top of the bolus. This is a characteristic feature of the vibration manufacturing process of the first aspect of the present invention. The second region may represent up to 1 % of the total weight of the bolus.

The inventors have found that such a structure enables two or more boluses to be easily bound to each other by the second region. This may be achieved by physically urging the second regions of two or more boluses together after step b) when the binder is still soft, or after step c) (which will require re-melting of the binder). Conventional bolus moulds can be substantially tubular; closed at the bottom by a rounded (e.g. convex/domed) base. A bolus formed in such a mould will be substantially tubular and have a rounded end (which is the first end of the bolus to be administered to the animal) opposed to a flat end (which would be the end that includes the second region). Two such boluses may be joined at the flat end, thereby providing a bolus composition with two rounded ends. After administration, the joined boluses may separate in the animal's rumen/reticulum at the binder joint and normal erosion of the intra-ruminal device occurs, assisted by erosion caused by collisions between the boluses.

Thus, in the present invention, there is provided a bolus composition comprising a plurality of the boluses described above, wherein the plurality of boluses are joined at the second region. Preferably the composition comprises two boluses joined to each other at their respective second regions.

Preferably the bolus (including the joined boluses or bolus compositions) is manufactured according to the process of the first aspect of the present invention.

In a further aspect of the present invention, there is provided a bolus as described above (including the joined boluses or bolus compositions) for use in a method of treatment. Such boluses may be used to treat disorders caused by nutrient deficiencies, when the bolus includes, as an active agent, the nutrient that is deficient in the diet of the animal to be treated. Preferably such a bolus includes a densifier which comprises zinc, which may be used in a method of treating facial eczema in ruminant animals. Facial eczema may be caused by sporidesmin, a mycotoxin from the spores of *Pithomyces chartarum.*

In yet a further aspect of the present invention there is provided a method for treating facial eczema in ruminant animals. The method involves administering to a ruminant animal the boluses described above (including the joined boluses or bolus compositions), which include an amount of zinc effective for treating facial eczema in a ruminant animal. When used to treat cattle and sheep, it is preferred that the amount of zinc administered by the bolus or boluses per day is 20 mgZn/kg live weight of animal to be treated (1 or more, but preferably less than 4, boluses administered together to achieve the aforementioned dosage regime). When used to administer iodine, the bolus or boluses preferably administer 0.5 to 2mg/kg of dry matter intake (i.e. feed), and to administer copper the bolus or boluses preferably administer 9-12mg/kg of dry matter intake. When used to administer biotin, the bolus or boluses preferably administer 20mg biotin per day.

### Detailed Description of the Invention

The present invention will now be described, by way of example, with reference to the accompanying drawings, in which:-
Figure 1 shows a box plot in which the weight loss (g) of boluses produced by the present invention (pour + agitate) and conventional methods (mix + compress) over a 14 day period is presented.
Figure 2 shows a chart of faecal zinc loss over time from animals administered with boluses according to the present invention, and according to conventional methods. Compressions 1, 2 and 3 were at 2 bar or less. The Compression Only readings were for a method that involved compressions of more than 10 bar.
Figure 3 shows linear release profiles for boluses that contain one or more trace elements, that were prepared according to the present invention and, that were tested in rumen fistulated cattle.

### EXAMPLE 1

The following example describes the result of an extrusion process for making boluses from 10% paraffin wax (MP 56°C) and 90% zinc.

The extruder was fitted with a multi-functional screw design which offered a range of possible feeding and venting options. The procedure used throughout was first to melt the wax in the extruder at about 1 kg/hr and 90 rpm using applied temperatures of 58° C. The zinc powder was then introduced at about 6kg/hr and progressively increased in order to observe the processing behaviour and extrudate texture on exit from the die adapter. The die plate was only fitted when the extrudate appeared to be adequately fluid to ensure uniform flow.

In a first trial the extrudate was initially a soft paste which thickened with increasing zinc content as expected. However, before the target of 90% content was reached, a loud rumbling noise was heard from the extruder together with large torque variations. The most likely cause of this was dry frictional rubbing and to minimise the effect a single screw side feeder was fitted to the extruder to allow the downstream addition of the zinc powder after the wax had been melted. At first, this set-up appeared to be stable with a steady extruder torque of 10% and so the die plate was fitted together with a die pressure transducer. On restart, the extruder torque began to vary indicating instability and after a few minutes the rumbling noise returned and the extruder stalled due to excessive torque demand (>100%). The extruder could not be restarted and so the clam-shell barrel was opened for cleaning. There was no indication of die pressure.

The next trial involved removing the side feeder and metering the zinc into the molten wax through an opened vent port on the top of the extruder barrel. This caused some problems with material build-up in the port but the extruder was kept running by cleaning with a plastic scraper. Using the 6mm strand die as before the extrudate flow was uniform when the paste was soft but with increments to the zinc content the flow became more irregular; ultimately causing the extruder to stall again. This time, when the die adapter was removed a very hard, zinc-rich layer was found on the extruder discharge screws. When this was chipped off, the extruder could be restarted without problem. Clearly, a build-up of this material over only a very short length was capable of over-torquing the extruder. The die adapter was refitted but without the die plate. A dry paste extrudate formed without problem with an estimated 85% zinc level. However, by partially blocking off the die adapter orifice with a brass scraper, the loud rumbling noise and high torque swings returned but could be relieved simply by removing the scraper.

The result showed that the formulation would not tolerate such pressure in extrusion. The most likely explanation for this extrusion behaviour is that the molten wax / zinc powder mixture is like wet sand which when squeezed will remove the water and leave, in effect, a solid zinc phase which simply compacts together sufficiently to stall the extruder.

### EXAMPLE 2

The following example describes a process according to the present invention:-
**Formulation Details** 60gm Cattle Bolus

| **Ingredient name (common/chemical)** | **CAS number** | **Concentration (%w/w)** | **Function** |
|---|---|---|---|
| Zinc | 7440-66-6 | 88.00 | Active |
| Parrifin Wax with melting temperature of 46°C, viscosity at 100 °C 2.7 - 10 mm²/s | 8002-74-2 | 11.94 | Excipient |
| Sunflower Oil | 8001-21-6 | 0.06 | Excipient |

**Zinc metal powder CAS-no. (7440-66-6)**
Sieve size

| | | |
|---|---|---|
| >250 Microns, +60 Mesh | 3% max | Sieve test |
| >150 Microns, +100 Mesh | 6% max | Sieve test |
| >106 Microns, +140 Mesh | 12-32% | Sieve test |
| >75 Microns, +200 Mesh | 25-50% | Sieve test |
| >45 Microns, +325 Mesh | 25-45% | Sieve test |
| <45 Microns, -325 Mesh | 20% max | Sieve test |

**Sunflower Oil (Helianthus Annuus Seed Oil) - CAS-no. (8001-21-6))**
Nutrient Specifications:-

| | | |
|---|---|---|
| Free fatty acids | 0.1% max | Ph. Eur. 2.4.22 (A) |
| Palmitic acid (C16) | 5-8% | Ph. Eur. 2.4.22 (A) |
| Stearic acid (C18) | 4-7% | Ph. Eur. 2.4.22 (A) |
| Oleic (C18:1) | 18-35% | Ph. Eur. 2.4.22 (A) |
| Linoleic (C18:2) | 56-70% | Ph. Eur. 2.4.22 (A) |
| Linolenic (C18:3) | 1% max | Ph. Eur. 2.4.22 (A) |

### Manufacturing method

The method used for making 30g Sheep and 60g/100g Cattle Zinc Boluses is as follows.

Waxes and sunflower oil are melted in a manufacturing drum, and zinc metal powder is preheated in a hopper (to about 60 °C or just above the melting point of the binders being used). The machine is set up as per the type of bolus required. Zinc is dispensed into pre-heated tubes (moulds) after which wax is dispensed into the tube. The wax soaks through the zinc powder and the mixture is agitated first then gently compressed (less than 2 bar) before being allowed to cool a little before being ejected from the tube and laid on cooling trays in order to harden.

### Machine Description

The machine is pneumatically actuated and PLC controlled. It is free standing and powered via a three phase 415V 50 Hz supply and an 80psi air supply.

Sited on the machine is a servo motor. One of two 'moulding drums' is attached to the motor. The drums consist of 60 stainless steel tubes. Fitted inside the tubes are brass inserts fitted with a plastic seal. The boluses are moulded inside these tubes. The drum is indexed into position via the servo motor.

Sited around the machine are various stations that are used to produce the Bolus :-
- Pre heat the tubes
- dispense zinc into the tubes
- dispense wax into the tubes
- supplementary heat to the tubes
- agitate the zinc/wax in the tube
- compact the zinc/wax in the tube
- cool the tubes
- eject the bolus
- return the inserts in the tube to the correct position in the tube

The machine has a HMI, this is used to set various operating parameters required for the production of the Bolus.

The machine also has a number of PID controllers, used to set the temperature of the various heaters

### (i) PRE-HEAT STATION

The drum tubes are pre-heated via a large 'air heater'. Compressed air at 3 cfu (max 6 cfu) is passed through a heated element, the hot air (250°C - 400°C) is directed via pipes into the stainless steel tubes. The heat is controlled via a PID controller.

### (ii) ZINC DISPENSE STATION

The Zinc is loaded into a hopper. The hopper is heated with a series of band heaters along the bottom stem. The heaters are controlled via PID controllers to ensure the zinc is heated to a temperature that is just above the melting point of the wax binders. A pre-set charge of heated Zinc is dispensed into the pre-heated tube. The moulding drum then indexes to the wax filling station and the next charge of heated Zinc is introduced (and so on). The charge of zinc is adjusted according to the size of bolus desired.

### (iii) WAX DISPENSE STATION

Pre-determined ratios of waxes are loaded in shredded form into a heated drum. The heaters are controlled via PID controllers.. The waxes are melted into liquid form in the drum. This liquid wax is also stirred during operation to ensure an even mix of the different types of waxes.

The drum is connected to a Peristaltic pump housed in a heated chamber. The chamber heaters are controlled via a PID controller. The pump is set with different programs depending upon how much wax is required to be dispensed according to the differing bolus sizes.

The pump is 'told' to dispense the wax into the stainless steel tubes as part of the indexing of the moulding drum.

### (iv) EXTRA HEAT STATION

Air process heaters can be positioned to add extra heat to different sections of the drum tubes if required, depending on the size of the bolus and the melting point of the waxes.

### (v) AGITATE STATION

A pneumatic cylinder is activated a number of times to agitate the zinc/wax mixture by dropping a rod (plunger) directly onto the molten mixture that results a small displacement of the mixture. The rod covers no more than 50% of the surface area of the molten mixture. Indeed displacement of the slurry around the edges of the impactor is desirable. The plunger on the end of the cylinder is heated to prevent wax mixture sticking to it. This process is repeated, a typical cycle is 6 where the rod is in contact with the molten mixture for 20 ms

Alternatively, single impact vibration, with a maximum frequency once every 3 seconds, can be applied directly to the metal slurry in the mould. This is not compression in the conventional sense and imposes a 2 bar or less pressure of the contents of the mould.

### (vi) COMPACT STATION

Two pneumatically activated cylinders will raise and lower to compact the zinc/wax mixture in the drum tube, but less than 2 bar pressure is applied. The plunger on the end of the lowering cylinder is heated to prevent wax sticking to it. The plunger is typically in contact with the molten material for no more than 1000 ms

### (v) TUBE COOLING

A set of fans are located around the drum to cool the zinc/wax mixture. Depending on the Bolus being produced, these can be turned on and off individually.

### (vi) EJECT BOLUS STATION

A pneumatically activated cylinder will be rise from under the table to eject the Bolus from the tubes, another will reset the insert to allow the process to be repeated.

### Finished Product Specifications

### (1) 60gm Zinc Bolus

| **TEST** | **METHOD** | **SPECIFICATION** |
|---|---|---|
| Appearance | Visual | 1 end flat & smooth, 1 end domed |
| Bolus surface appearance | Visual & sensory | Smooth, grey no pitting/dimples or airholes to be < 3mm diameter |
| Odour | Olfactory | Nil |
| Length of Bolus | Measure length of boluses using callipers | 34mm +/- 5% |
| Weight | ***Individually weigh 20 boluses. Of the 20 no more than 2 weights deviate from the average by > +*/*- 5% No weight deviates by > 10%*** | 60g+/-5% |
| Weight per mm | Calculate from weight & length measurements | 1.8g/mm +/- 5% |
| Disintegration test | Use disintegration tester as described in Ph. Eur | Boluses should soften but not swell & lose integrity when immersed in water at 37°C for a minimum of 2 hours |
| Content: Zinc | Zinc Assay | 85.5-90.5% |

### EXAMPLE 3

The following compares the results of boluses made with 88% zinc metal powder and 12 % pure paraffin wax (MP 46°C) either by
- heating and mixing the components, transfer to a mould and compression with a hydraulic press to a pressure of greater than 10 bar, or
- by pouring the wax over the metal powder, agitation then compression with a pressure of less than 2 bar (i.e. according to example 2).

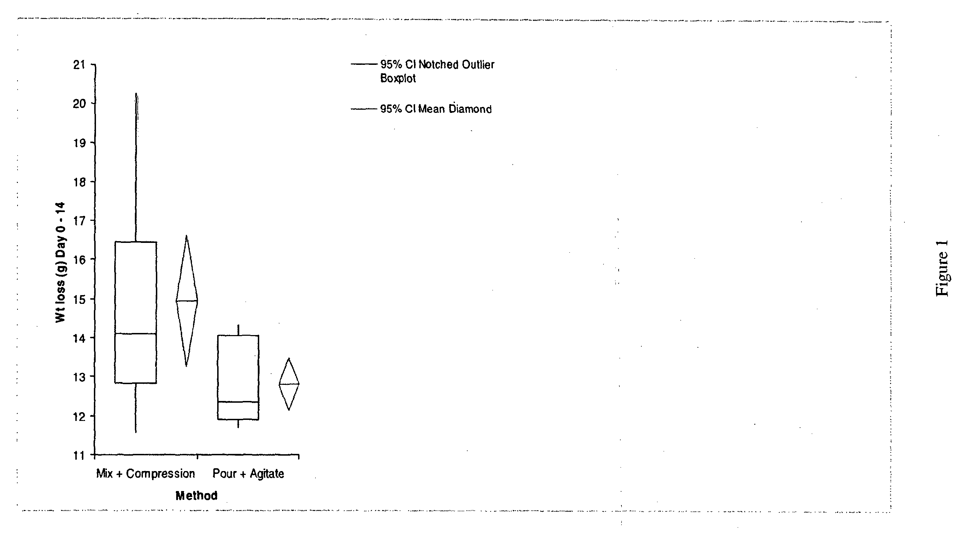

Results are shown in Figure 1

Twleve boluses from each method were tested in 3 cattle over 14 days.

| ***Wt loss (g) 0 - 14 Days by Method*** | ***n*** | ***Mean*** | ***Range*** | ***SE*** | ***C of V*** |
|---|---|---|---|---|---|
| Pour + Agitate | 12 | 12.82 | 11.7 to 14.4 | 0.31 | 8% |
| Mix + Compression | 12 | 14.94 | 11.6 to 20.3 | 0.76 | 18% |

The results show that despite identical materials being used there was less variation in release rates of the boluses made by agitation. Evidence of this is shown by:
- The coefficient of variation. For the agitate method it was 8% compared to 18% for the compression only method.
- The range of weight loss. For the compression only method it ranged from 11.6g to 20.3g compared to 11.7g to 14.4g. As a consequence, the standard error of the compression method was double.

The mean weight loss was significantly less (P<0.05) for the agitation method.

### EXAMPLE 4

The following is an example of the same machine being used to produce some zinc boluses (both with the same basic formulation, 88% zinc 12% wax with two different melting points (46°C and 56°C) in a ratio of 60:40). The processes for making the boluses studied in this example differ by the fact that one process does not involve agitation (i.e. vibration) but does involve compression to greater than 10 bar, whilst the other process involves agitation and compression to less than 2 bar. Agitation has a marked effect in reducing the variation in the release profile of the bolus, particularly in the first 14 days.

| ***Day 7 Zn mg*/*kg by Method*** | ***n*** | ***Mean*** | ***Range*** | ***SE*** | ***C of V*** |
|---|---|---|---|---|---|
| Pour +Agitate + Compress 1 | 5 | 144.00 | 100.0 to 210.0 | 18.60 | 29% |
| Pour + Compression only | 21 | 381.81 | 65.0 to 1100.0 | 67.89 | 82% |

| ***Day 14 Zn mg*/*kg by Method*** | ***n.*** | ***Mean*** | ***Range*** | **SE** | ***C of V*** |
|---|---|---|---|---|---|
| Pour +Agitate + Compress 1 | 5 | 136.00 | 100.0 to 180.0 | 15.68 | 26% |
| Pour + Compression only | 21* | 209.81 | 16.0 to 430.0 | 23.37 | 51% |

| ***Day 21 Zn mg*/*kg by Method*** | ***n*** | ***Mean*** | ***Range*** | ***SE*** | ***C of V*** |
|---|---|---|---|---|---|
| Pour +Agitate + Compress 1 | 5 | 168.00 | 110.0 to 240.0 | 25.96 | 35% |
| Pour + Compression only | 21 | 184.19 | 38.0 to 430.0 | 17.27 | 43% |

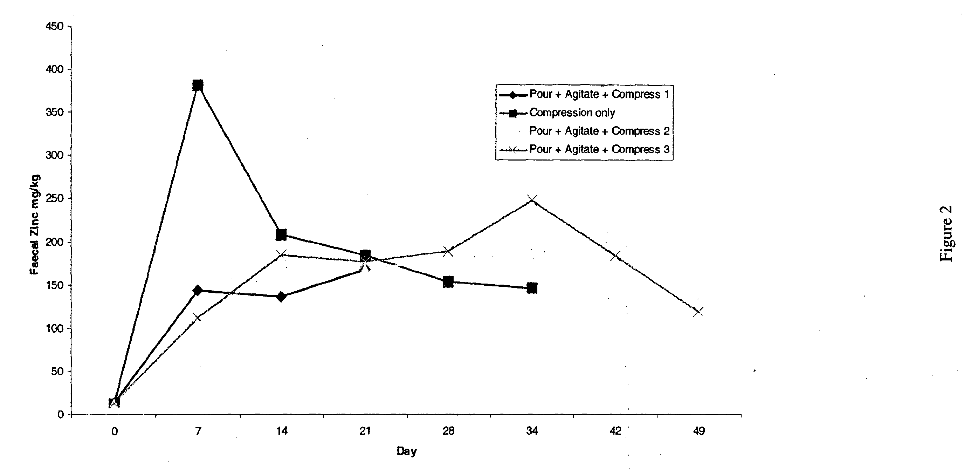

The results above are presented also in Figure 2 to graphically illustrate the release profile. Two other profiles are also added. Pour + Agitate + Compression Groups 2 and 3 show the release profile of 88% zinc but with slightly different ratios of wax binders. Compression 2 and 3 is less than 2 bar. Group 2 has a two wax with two different melting points (46°C and 56°C) in a ratio of 80:20. Group 3 is 100% paraffin wax with MP 46°C). This batch had the same basic formulation, 88% zinc 12% wax.

In contrast to the compression only variant that has a marked spike in zinc release.

### EXAMPLE 5

A comparison of the physical properties from two manufacturing methods. The following data compares 100g zinc boluses (all with the same basic formulation, 88% zinc 12% wax as described in earlier examples) made by either
- pre-mixing and compression with a hydraulic press at a pressure of greater than 10 bar or
- pour + agitate + compress (less than 2 bar)

The analyses tested two aspects of the finished product: the final length of the bolus and the weight per mm (g/mm). As the initial charge of material is the same (100g) and the diameter the same (26mm), both parameters give and indication of the degree of compaction of the bolus.

Interestingly there is no significant difference in the physical parameters of the two methods despite the fact that the hydraulic press applies over 10 bar compared to the pneumatic compaction on the machines that is regulated to 2 bar max.
a) ANOVA of the length (mm) of boluses produced by two different manufacturing methods.

| Length by thod | n | Mean | SE | Pooled SE | SD |
|---|---|---|---|---|---|
| Mix + Compression | 20 | 55.3115 | 0.2593 | 0.1615 | 1.160 |
| Pour + Agitate + Compress | 60 | 55.420 | 0.0654 | 0.0932 | 0.506 |

| Source of variation | squares | DF | Mean square | F statistic | p |
|---|---|---|---|---|---|
| Method | 0.165 | 1 | 0.165 | 0.32 | 0.5749 |
| Residual | 40.671 | 78 | 0.521 | | |
| Total | 40.836 | 79 | | | |

b) ANOVA of the weight per mm (g/mm) of boluses produced by two different manufacturing methods.

| Weight/ml by Method | n | Mean | SE | Pooled SE | SD |
|---|---|---|---|---|---|
| Mix + Compression | 20 | 1.809 | 0.0085 | 0.0060 | 0.038 |
| Pour + Agitate + Compress | 60 | 1.814 | 0.0029 | 0.0035 | 0.022 |

| Source of variation | Sum squares | DF | Mean square | F statistic | p |
|---|---|---|---|---|---|
| Method | 0.000 | 1 | 0.000 | 0.59 | 0.4437 |
| Residual | 0.057 | 78 | 0.001 | | |
| Total | 0.057 | 79 | | | |

### EXAMPLE 6

The addition of additional actives to the densifier.

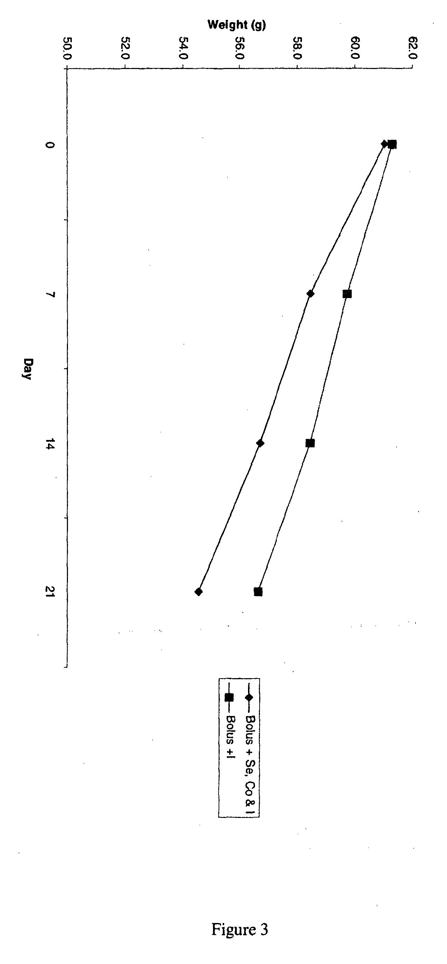

The pour + agitate + compress method according to example 2 above works also for a range of trace element additives. Figure 3 illustrates, desirable linear release profiles for boluses containing one or more trace elements tested in rumen fistulated cattle,
Pour + Agitate + Compress bolus (60g)

| | | ***Day*** | | | |
|---|---|---|---|---|---|
| ***Cow*** | ***Batch*** | ***0*** | ***7*** | ***14*** | ***21*** |
| 6 | 1058 | 61.0 | 58.9 | 57.4 | 55.4 |
| 6 | 1058 | 60.9 | 58.6 | 57.0 | 54.9 |
| 11 | 1058 | 61.6 | 58.5 | 56.9 | 55.4 |
| 11 | 1058 | 60.8 | 59.2 | 57.6 | 55.2 |
| 17 | 1058 | 60.7 | 58.0 | 55.9 | 53.0 |
| 17 | 1058 | 61.2 | 57.5 | 55.4 | 53.4 |
| ***Av*** | ***1058*** | ***61.0*** | ***58.5*** | ***56.7*** | ***54.6*** |
| 35 | 2058 | 61.4 | 60.1 | 58.8 | 56.9 |
| 35 | 2058 | 61.2 | 60.1 | 58.8 | 57.0 |
| 54 | 2058 | 61.3 | 59.3 | 58.2 | 56.2 |
| 54 | 2058 | 61.4 | 59.5 | 58.0 | 56.4 |
| ***Av*** | ***2058*** | ***61.3*** | ***59.8*** | ***58.5*** | ***56.6*** |

| | | | | | |
|---|---|---|---|---|---|
| BATCH 1058= 60g Zinc Boluses made with 1% Oil (50:50 Wax) + Cobalt Carbonate, Sodium Selenate & Calcium lodate BATCH 2058 = 60g Zinc Boluses made with 1% Oil (50:50 Wax) + Calcium lodate | | | | | |

### Example 7

The table below shows the results of a study of 10 boluses manufactured according to the present invention, and 10 boluses manufactured in the same manner but without vibration.

All boluses were manufactured with the same basic formulation (88%.Zinc, 12%.Wax) as discussed in previous examples and in accordance with the same basic protocol.

It can be seen that vibration reduces the overall length of the bolus to 32.3 mm, compared to 33.9 mm for boluses produced with vibration. This equates to a 4.6% reduction in the length of the bolus by changing the packing geometry of the particles, whilst eliminating the requirement for compression and significantly increases the speed of manufacture.

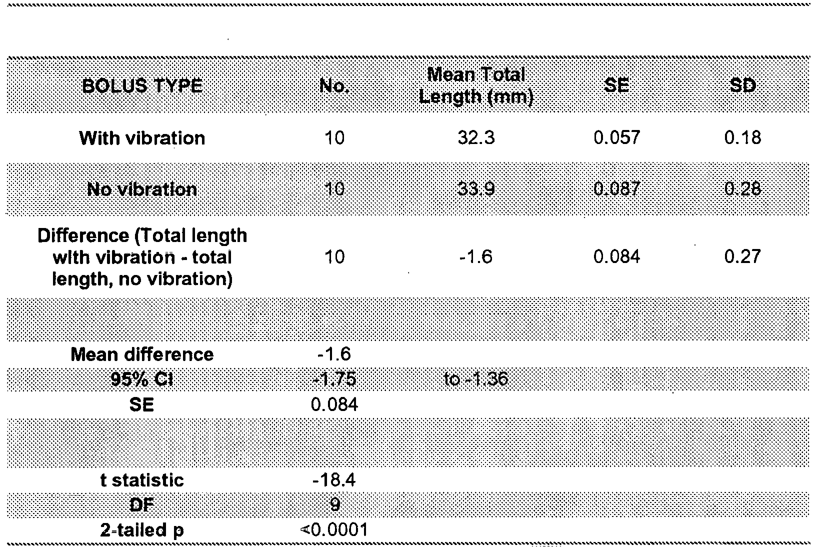

## Claims

1. A process for manufacturing a bolus, wherein the process comprises the steps of:-
(a) introducing a densifier and a binder into a mould without the densifier and binder having been mixed prior to step a;
(b) mixing the contents of the mould, which comprise the densifier and the binder, by vibration;
(c) solidifying the contents of the mould so as to form a bolus, wherein the binder comprises a resin or a wax and is fluid during steps a. and b.

2. A process as claimed in claim 1, wherein the densifier and binder are separately, sequentially or simultaneously introduced to the mould.

3. A process as claimed in claim 1 or claim 2, wherein the densifier;
(a) comprises a metal or metal compound.
(b) is zinc, and/or;
(C) is particulate.

4. A process as claimed in any of the preceding claims, wherein 5% or less of the particles have a diameter greater than 150 µm, from 50 to 80% of the particles have a diameter from 45 to 150 µm, and less than 40% of the particles have a diameter of less than 45 µm.

5. A process as claimed in any of the preceding claims, wherein from 80 to 95 % by weight of the bolus is densifier.

6. A process as claimed in any of the preceding claims, wherein the binder comprises paraffin wax; carnauba wax; beeswax, or the like, or any combination thereof.

7. A process as claimed in any of the preceding claims, wherein the binder is molten during steps a and b, and solidified during step c on cooling.

8. A process as claimed in any claims 1 to 7, wherein the contents of the mould is not compressed.

9. A process as claimed in any of the preceding claims, wherein the amount of binder introduced in step a is the minimum amount of binder required to cover all of the densifier in the mould prior to step b.

10. A process as claimed in any of the preceding claims, wherein step a. includes the introduction of a therapeutic agent to the mould and the therapeutic agent comprises :- selenium, cobalt, iodine, manganese, copper, zinc, vitamins, lipids, biotin, amino acids, anthelmintic agents, antibacterial agents, growth promoters, hormones, coccidiostats,
or flukicides, or any combination thereof.

11. A bolus composition comprising a plurality of the boluses manufactured by the process claimed in any of the preceding claims, wherein a first region of each bolus comprises densifier homogeneously mixed throughout the binder and a second region of each bolus comprises binder and in which densifier is absent, the plurality of boluses being joined at the second region.

12. A bolus composition as claimed in claim 11, wherein the second region forms a film on a portion of the surface of the bolus

13. A bolus composition as claimed in claim 12, wherein the film is formed on a single substantially flat surface of each bolus.

14. A bolus composition as claimed in any of claims 11 to 13, further comprising :- selenium, cobalt, iodine, manganese, copper, zinc, vitamins, lipids, biotin, amino acids, anthelmintic agents, antibacterial agents, growth promoters, hormones, coccidiostats, or flukicides, or any combination thereof.

15. A bolus composition as claimed in any of claims 11 to 14, wherein the densifier comprises zinc metal powder and the bolus composition is for use in a method of treatment.

## Patentansprüche

1. Ein Verfahren zum Herstellen eines Bolus, wobei das Verfahren die folgenden Schritte beinhaltet:
(a) Einführen eines Verdichters und eines Bindemittels in eine Form, ohne dass der Verdichter und das Bindemittel vor Schritt a vermischt worden sind;
(b) Vermischen des Inhalts der Form, wobei dieser den Verdichter und das Bindemittel beinhaltet, durch Vibration;
(c) Verfestigen des Inhalts der Form, um einen Bolus zu bilden, wobei das Bindemittel ein Harz oder ein Wachs beinhaltet und während der Schritte a. und b. fluide ist.

2. Verfahren gemäß Anspruch 1, wobei der Verdichter und das Bindemittel getrennt, nacheinander oder gleichzeitig in die Form eingeführt werden.

3. Verfahren gemäß Anspruch 1 oder Anspruch 2, wobei der Verdichter;
(a) ein Metall oder eine Metallverbindung beinhaltet.
(b) Zink ist und/oder;
(c) partikelförmig ist.

4. Verfahren gemäß einem der vorhergehenden Ansprüche, wobei 5 % oder weniger der Partikel einen Durchmesser von mehr als 150 µm aufweisen, 50 bis 80 % der Partikel einen Durchmesser von 45 bis 150 µm aufweisen und weniger als 40 % der Partikel einen Durchmesser von weniger als 45 µm aufweisen.

5. Verfahren gemäß einem der vorhergehenden Ansprüche, wobei 80 bis 95 Gew.-% des Bolus der Verdichter ist.

6. Verfahren gemäß einem der vorhergehenden Ansprüche, wobei das Bindemittel Paraffinwachs, Carnaubawachs, Bienenwachs oder dergleichen oder eine beliebige Kombination davon beinhaltet.

7. Verfahren gemäß einem der vorhergehenden Ansprüche, wobei das Bindemittel während der Schritte a und b geschmolzen ist und dann während des Schrittes c beim Auskühlen verfestigt wird.

8. Verfahren gemäß einem der Ansprüche 1 bis 7, wobei der Inhalt der Form nicht komprimiert wird.

9. Verfahren gemäß einem der vorhergehenden Ansprüche, wobei die Menge an Bindemittel, die in Schritt a eingeführt wird, die Mindestmenge an Bindemittel ist, die erforderlich ist, um den gesamten Verdichter in der Form vor Schritt b zu bedecken.

10. Verfahren gemäß einem der vorhergehenden Ansprüche, wobei Schritt a das Einführen eines Therapeutikums in die Form einschließt und das Therapeutikum Folgendes beinhaltet: Selen, Kobalt, Jod, Mangan, Kupfer, Zink, Vitamine, Lipide, Biotin, Aminosäuren, Anthelmintika, antibakterielle Mittel, Wachstumsförderer, Hormone, Kokzidiostatika oder Flukizide oder eine beliebige Kombination davon.

11. Eine Bolus-Zusammensetzung, die eine Vielzahl der Boli beinhaltet, die durch das Verfahren gemäß einem der vorhergehenden Ansprüche hergestellt wurden, wobei ein erster Bereich jedes Bolus einen homogen in dem Bindemittel vermischten Verdichter beinhaltet und ein zweiter Bereich jedes Bolus ein Bindemittel beinhaltet und in dem der Verdichter abwesend ist, wobei die Vielzahl von Boli an dem zweiten Bereich verbunden ist.

12. Bolus-Zusammensetzung gemäß Anspruch 11, wobei der zweite Bereich einen Film auf einem Teilbereich der Oberfläche des Bolus bildet.

13. Bolus-Zusammensetzung gemäß Anspruch 12, wobei der Film auf einer einzelnen, im Wesentlichen flachen Oberfläche jedes Bolus gebildet ist.

14. Bolus-Zusammensetzung gemäß einem der Ansprüche 11 bis 13, die ferner Folgendes beinhaltet: Selen, Kobalt, Jod, Mangan, Kupfer, Zink, Vitamine, Lipide, Biotin, Aminosäuren, Anthelmintika, antibakterielle Mittel, Wachstumsförderer, Hormone, Kokzidiostatika oder Flukizide oder eine beliebige Kombination davon.

15. Bolus-Zusammensetzung gemäß einem der Ansprüche 11 bis 14, wobei der Verdichter Zinkmetallpulver beinhaltet und die Bolus-Zusammensetzung der Verwendung in einer Behandlungsmethode dient.

## Revendications

1. Un procédé de fabrication d'un bolus, le procédé comprenant les étapes suivantes :
(a) introduire un densificateur et un liant dans un moule sans que le densificateur et le liant n'aient été mélangés avant l'étape a ;
(b) mélanger le contenu du moule, lequel comprend le densificateur et le liant, par vibration ;
(c) solidifier le contenu du moule de façon à former un bolus, dans lequel le liant comprend une résine ou une cire et est fluide durant les étapes a et b.

2. Un procédé tel que revendiqué dans la revendication 1, dans lequel le densificateur et le liant sont introduits séparément, séquentiellement ou simultanément dans le moule.

3. Un procédé tel que revendiqué dans la revendication 1 ou la revendication 2, dans lequel le densificateur ;
(a) comprend un métal ou un composé métallique.
(b) est du zinc, et/ou ;
(c) est une matière particulaire.

4. Un procédé tel que revendiqué dans n'importe lesquelles des revendications précédentes, dans lequel 5 % ou moins des particules ont un diamètre supérieur à 150 µm, de 50 à 80 % des particules ont un diamètre allant de 45 à 150 µm, et moins de 40 % des particules ont un diamètre de moins de 45 µm.

5. Un procédé tel que revendiqué dans n'importe lesquelles des revendications précédentes, dans lequel le densificateur représente de 80 à 95 % en poids du bolus.

6. Un procédé tel que revendiqué dans n'importe lesquelles des revendications précédentes, dans lequel le liant comprend de la cire de paraffine ; de la cire de carnauba ; de la cire d'abeille, ou analogue, ou n'importe quelle combinaison de celles-ci.

7. Un procédé tel que revendiqué dans n'importe lesquelles des revendications précédentes, dans lequel le liant est fondu au cours des étapes a et b, et solidifié au cours de l'étape c en refroidissant.

8. Un procédé tel que revendiqué dans n'importe quelles revendications 1 à 7, dans lequel le contenu du moule n'est pas comprimé.

9. Un procédé tel que revendiqué dans n'importe lesquelles des revendications précédentes, dans lequel la quantité de liant introduite à l'étape a est la quantité minimum de liant requise pour recouvrir la totalité du densificateur dans le moule avant l'étape b.

10. Un procédé tel que revendiqué dans n'importe lesquelles des revendications précédentes, dans lequel l'étape a comporte l'introduction d'un agent thérapeutique dans le moule et l'agent thérapeutique comprend : du sélénium, du cobalt, de l'iode, du manganèse, du cuivre, du zinc, des vitamines, des lipides, de la biotine, des acides aminés, des agents anthelminthiques, des agents antibactériens, des activateurs de croissance, des hormones, des anticoccidiens, ou des douvicides, ou n'importe quelle combinaison de ceux-ci.

11. Une composition de bolus comprenant une pluralité des bolus fabriqués par le procédé revendiqué dans n'importe lesquelles des revendications précédentes, dans laquelle une première région de chaque bolus comprend un densificateur mélangé de façon homogène dans le liant et une deuxième région de chaque bolus comprend du liant mais pas de densificateur, la pluralité de bolus étant jointe au niveau de la deuxième région.

12. Une composition de bolus telle que revendiquée dans la revendication 11, dans laquelle la deuxième région forme un film sur une portion de la surface du bolus.

13. Une composition de bolus telle que revendiquée dans la revendication 12, dans laquelle le film est formé sur une surface unique substantiellement plate de chaque bolus.

14. Une composition de bolus telle que revendiquée dans n'importe lesquelles des revendications 11 à 13, comprenant de surcroît : du sélénium, du cobalt, de l'iode, du manganèse, du cuivre, du zinc, des vitamines, des lipides, de la biotine, des acides aminés, des agents anthelminthiques, des agents antibactériens, des activateurs de croissance, des hormones, des anticoccidiens, ou des douvicides, ou n'importe quelle combinaison de ceux-ci.

15. Une composition de bolus telle que revendiquée dans n'importe lesquelles des revendications 11 à 14, dans laquelle le densificateur comprend de la poudre métallique de zinc et la composition de bolus est destinée à être utilisée dans une méthode de traitement.
